Europäisches Patentamt

European Patent Office

Office européen des brevets

(11) Publication number: **0 077 063 B1**

(12) **EUROPEAN PATENT SPECIFICATION**

(45) Date of publication of patent specification: **16.03.88**

(51) Int. Cl.⁴: **A 61 K 45/02** // (A61K45/02, 31:365)

(21) Application number: **82109391.1**

(22) Date of filing: **11.10.82**

(54) Interferon-containing compositions and the use of these compositions in the treatment of herpetic infections, pre-malignant skin lesions, skin malignancies and psoriasis.

(30) Priority: **13.10.81 US 311035**
**14.06.82 US 388260**

(43) Date of publication of application:
**20.04.83 Bulletin 83/16**

(45) Publication of the grant of the patent:
**16.03.88 Bulletin 88/11**

(84) Designated Contracting States:
**BE CH DE FR GB IT LI NL SE**

(56) References cited:
**FR-A-2 266 740**
**FR-A-2 266 741**
**US-A-4 020 183**
**US-A-4 053 582**
**US-A-4 061 538**
**US-A-4 139 630**
**US-A-4 147 803**
**US-A-4 184 917**

The file contains technical information submitted after the application was filed and not included in this specification

(73) Proprietor: **Exovir, Inc.**
**10 Cutter Mill Road**
**Great Neck New York 11021 (US)**

(72) Inventor: **Asculai, Samuel S.**
**2077 Central Dr. N.**
**East Meadow New York 11554 (US)**
Inventor: **Rapp, Fred**
**2 Laurel Ridge Road**
**Hershey Pennsylvania 17033 (US)**
Inventor: **Miller, Daniel G.**
**6 Fox Meadow Road**
**Scarsdale New York 10583 (US)**

(74) Representative: **Vossius & Partner**
**Siebertstrasse 4 P.O. Box 86 07 67**
**D-8000 München 86 (DE)**

(56) References cited:
**CHEMICAL ABSTRACTS, vol.69, no.11, September 9, 1968, page 3995, abstract no.42705p, Columbus, Ohio (US) T. C. MERIGAN et al.: "Interferon-stimulating and in vivo antiviral effects of various synthetic anionic polymers"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to pharmaceutical compositions for treating herpes simplex viral infections in humans. More particularly, this invention relates to pharmaceutical compositions containing an effective amount of human interferon and an antiviral nonionic surface active agent for topical application.

Infections by herpes simplex virus are extremely common in humans. The virus is transmitted by direct contact with infected individuals and it is estimated that more than half the human population has been infected by herpes simplex virus at one time or another.

Once an individual is infected, the virus manifests itself as sores or lesions are various parts of the body. Herpes labialis is the most clinical form of herpes simplex virus infection. The symptoms include inflammation of the mouth and gums as well as mouth eruptions (cold sores). The cold sores are often painful and unsightly.

In another common clinical form, herpes genitalis, eruptions occur in the gential area. Herpes genitalis is a serious problem. It often causes severe psychological and social problems in affected individuals. It can be fatal to patients with deficient immune systems. In addition, it presents the risk of infections to the newborns of infected mothers, often a fatal disease to the infant.

Herpes simplex virus may also infect the eye producing acute keratitis, and may infect the central nervous system resulting in severe encephalitis.

Although it was once thought that there was only one type of herpes simplex virus, it is now known that there are two major types of the virus. Herpes simplex virus type 1 (hereinafter HSV-1) is usually associated with herpes labialis. Herpes simplex virus type 2 (hereinafter HSV-2) is usually associated with herpes genitalis. However, HSV-1 has been isolated in some instances from genital lesions, while HSV-2 has been isolated from lesions on parts of the body other than the genitals. In some reports, HSV-1 has been linked to lip cancer while HSV-2 has been linked to cervical and vulvar cancer.

Following the initial infection with either HSV-1 or HSV-2 lesions may or may not appear. However, the virus does not die but continues to reside in a latent form in the nerve ganglia. Recurrent attacks may occur throughout life in response to non-specific stimuli, for example, in response to changes in body temperature, stress, ultraviolet radiation or hormonal changes. It is believed by some that recurrent attacks occur when the host's immune system is suppressed. At that time, virus latent in the ganglia travels along the nerve cells without damaging them until it emerges from the ends of the nerves. As it emerges, the virus infects the adjacent cells and begins to multiply.

Viral multiplication takes place in two stages eventually resulting in a lesion. In the first stage, known as the prodromal stage, the number of viral particles increases until a first maximum is reached. During this stage, the patient may feel a tingling sensation at the site of viral multiplication. The number of viral particles decreases thereafter. About two to three days after the prodromal stage, the viral particles undergo a second stage of multiplication. In the second stage, number of viral particles increases until a second maximum, much larger than the first maximum, is reached. The second stage of multiplication causes the death of many cells and results in a lesion. Usually, the lesion heals within 10 to 14 days but in some cases the lesion can last much longer.

Many claims have been made for effective antiherpetic compositions. Undoubtedly, the benefits demonstrated by many of these compositions in *in vivo* tests may be attributed to a placebo effect. This effect may be as high as 50% for herpes simplex viral infections. One group of chemical agents which proved effective against herpes simplex virus in *in vitro* tests is the group comprising lipid-dissolving anionic and cationic surface active agents. The effectiveness of these agents has been attributed to their ability to dissolve the lipid-containing membrane which surrounds the nucleocapsid of both HSV-1 and HSV-2.

More recently, it has been discovered by one of the inventors herein along with others that nonionic surface active agents are also effective in reducing the infectivity of herpes simplex virus. For example, in Asculai, S.S., et al, *Antimicrobial Agents and Chemotherapy*, *13*, 686 (1978), it is reported that certain nonionic surface active agents rapidly reduced the infectivity of HSV-1 and HSV-2 *in vitro*. The nonionic surface active agents which inactivated the infectivity of herpes simplex virus were those possessing ether or amide linkages between the hydrophilic and the hydrophobic portions of the molecule.

See also U.S.—A—4,020,183 (Asculai et al) and U.S.—A—4,139,630 (Asculai et al). The therapeutic effect of these nonionic surfactants was also attributed to their ability to dissolve the lipid-containing envelope of herpes simplex virus. The nonionic surfactants were also reported to destroy partially the nucleocapsid of the virus. The nonionic surfactants find use as spermicides in vaginal contraceptives. Contraceptive formulations containing nonionic surfactants were also demonstrated to be effective against herpes simplex virus.

The treatment of herpes simplex virus infections by topical administration of surface active agents, whether anionic, cationic, or nonionic, does not produce entirely satisfactory results. The most beneficial results are obtained when the surfactant is applied during the prodromal stage before the lesion appears. However, it is often difficult to tell when the virus is in the prodromal stage of multiplication. Furthermore, the surface active agent is applied to the uppermost layer of infected cells. Because of the dilution effect, the cells below the uppermost layer do not receive sufficient amounts of the surface active agent to destroy the viral particles. Thus, no protection is provided for cells below the surface layer and herpes simplex virus continues to multiply in those cells below the surface layer.

Additionally, treatment with the antiviral surface active agent does not appear to reduce the frequency of recurrent attacks. It is believed, however, that recurrent attacks might be prevented if sufficient numbers of the multiplying viral particles are destroyed.

It would thus be desirable to provide a pharmaceutical composition which reduces the load of herpes simplex virus so that latency is not reestablished.

It would also be desirable to provide a pharmaceutical composition which prevents spread of the virus to cells below the surface layer.

Human interferon is known to protect cells against viral infections. Human interferon is produced by cells in reaction to the presence of specific inducers, such as viruses. It may be produced *in vivo* by the cells of living organisms, or it may be produced *in vitro* by cell cultures in response to the presence of the inducer. There are now known to be three main varieties of human interferon: leukocyte or α, fibroblast or β, and immune or γ interferon. There are also known to be several sub-varieties of human leukocyte and fibroblast interferon.

Human interferon is relatively nontoxic and nonantigenic in humans. It is also extremely effective against a broad spectrum of viruses, including herpes simplex virus, even at very low concentrations. Until the present, treatment of herpetic lesions with human interferon has proceeded along two main courses: a) medicinal induction of endogenic interferon in the patient, and b) administration of exogenic interferon to the patient.

For example, U.S.—A—4,053,582 (Stickl) discloses a method for treating herpes simplex infections in humans by administering attenuated fowl pox virus to the patient. The attenuated virus induces the patient to produce his own interferon. The herpetic lesions heal within a short time after induction.

U.S.—A—4,061,538 (Dorner et al) and 4,184,917 (Dorner et al) disclose a method of treating herpes simplex viral infections by administering structurally modified interferons to the patient. In these patents, the modified interferons are administered systemically to the patient.

Several published reports also disclose the treatment of herpetic dye infections by topical administration of human interferon. For example, see D. Naumann-Haefelin et al in *Infection and Immunity, 17*, 468 (1977) and B. R. Jones et al in *Lancet ii,* 128 (1976).

None of these disclosures suggests the treatment of herpes labialis and herpes genitalis in humans by administering topically an interferon-containing pharmaceutical composition. Moreover, none of these disclosures suggests the treatment of herpes simplex viral infections by administering topically a combination of human interferon and an antiviral nonionic surface active agent.

Basal cell carcinomas, which may appear as small, shiny, firm nodules or ulcerated crusted lesions on the skin, are generally treated by electrodesiccation and curettage, surgical excision or X-ray therapy. In about 5% of cases, recurrences occur. Such recurrences are treated typically with further excision or Moh's chemosurgery, i.e., microscopically controlled excision after chemical fixation of the tissue.

Squamous cell carcinoma arises from the malpighian cells of the epithelium. The tumor generally begins as a small red papule or plaque with a scaly or crusted surface. Subsequently, it becomes nodular. Eventually, the lesion ulcerates and invades the underlying tissue. As with basal cell carcinoma, squamous cell carcinoma is treated with electrodesiccation and curettage, surgical excision or X-ray therapy.

Precancerous keratotic lesions, actinic keratoses, are the frequent consequence of many years of over-exposure to sunlight. The keratoses are usually hard, and gray to dark in color. Such lesions are generally treated by topical application of fluorouracil (5-fluorouracil). However, if the lesions become malignant, topical application of fluorouracil is not recommended as the rate of recurrence is unacceptably high with such therapy.

Leukoplakia, hyperkeratinization of the oral mucosa, is generally believed to result from several factors, local or systemic, acting independently or in combination, such as: tobacco, alcohol, chronic irritation from such causes as cheek biting, ill-fitting dentures, sharp, broken or worn-down teeth or spicy foods, syphilis, vitamin deficiency, hormonal changes and *Candida albicans*. Leukoplakia lesions vary from a small, well-localized white patch to a diffuse area involving much of the oral mucosa, and from a smooth, flat, or slightly elevated, translucent white plaque to a thick, fissured papillomatous lesion that is firm to palpation. Treatment of leukoplakia generally comprises initial removal or elimination of any recognizable irritating factors, such as tobacco or faulty restoration or prosthetic devices, to chemical irritants, and total excision or cauterization of the localized lesions. Large lesions may be removed by a series of "stripping" operations. The patient should additionally be re-examined periodically and any lesions subsequently located biopsied because of the pre-cancerous nature of the disease. Additionally, leukoplakia may be involved in lesions of the vulva and anus. When involved with the vulva, leukoplakia is treated by total vulvectomy.

Psoriasis is characterized by skin lesions which are sharply demarcated, usually non-pruritic, erythematous papules or plaques covered with overlapping, silvery or slightly opalescent, shiny scales. The typical course of psoriasis is chronic remission and recurrence, which vary in frequency and duration. Psoriasis varies in severity from one or two lesions to a widespread dermatosis, to psoriatic arthritis or exfoliation. Treatment depends upon the extent and severity of the involvement. The simplest form of treatment, topical application of lubricants, keratolytics and corticosteroids, is initially employed. Systemic

3

antimetabolites should be used only in severe skin or joint involvement because of their potential adverse effects. Exposure to ultraviolet light has been found helpful in the treatment of psoriasis. Methotrexate taken orally is generally an effective treatment in severe disabling psoriasis which has not responded to topical therapy. However, because of the toxicity of methotrexate, hematological, renal and hepatic functions of the patient must be carefully monitored.

Herpes zoster is a virus-related, acute CNS infection involving primarily the dorsal root ganglia, and characterized by vesicular eruption and neuralgic pain in the cutaneous area supplied by peripheral sensory nerves arising in the affected root ganglia. Herpes zoster may be activated by local lesions or by systemic disease, among other reasons. Prodromal symptoms of chills and fever, malaise and GI disturbances may be present for three or four days before the distinctive features of the disease develop. On about the fourth or fifth day, characteristic crops of vesicles on an erythematous base appear, following the cutaneous distribution of one or more posterior root ganglia. There is no specific treatment for herpes zoster. Early systemic application of a corticosteroid, however, generally relieves pain.

Accordingly, it is an object of the present invention to provide novel pharmaceutical compositions containing a human interferon and an antiviral nonionic surface active agent for topical application which not only heal the lesions but may also reduce recurrent attacks, for treatment of malignant and premalignant skin lesions, herpes zoster and psoriasis, and for treatment of skin lesions produced by squamous cell carcinoma, basal cell carcinoma, actinic keratosis, leukoplakia, psoriasis and herpes zoster.

How these and other objects of this invention are achieved will become apparent in light of the accompanying disclosure and claims.

The pharmaceutical compositions of the present invention consist of $10^2$ to $10^8$ I.U. per dosage unit of a human interferon, an effective amount of an antiviral nonionic surface active agent generally 0.1% to 20% by weight and a physiologically acceptable carrier for topical application. Most preferably, a composition for treating herpes simplex viral infections comprises about $10^4$ to $10^6$ I.U. human leukocyte interferon, 1% to 5% by weight of a nonionic surface active agent having at least one ether or amide linkage, and a physiologically acceptable carrier.

The novel pharmaceutical compositions of the present invention contain effective amounts of a human interferon, an antiviral nonionic surface active agent, and a physiologically acceptable carrier. The pharmaceutical compositions may contain any of the known varieties of sub-varieties of human interferon. Thus, the composition may contain human leukocyte, fibroblast, or immune interferon. Additionally, the interferon may suitably be prepared by "classical" culture methods or by recombinant DNA methods. An effective dosage of human interferon for treating herpetic lesions in accordance with the practices of the present invention is $10^2$ to $10^8$, preferably $10^4$ to $10^6$, I.U.

Antiviral nonionic surface active agents are known in the art. These surfactants dramatically reduce the infectivity of HSV-1 and HSV-2 by damaging or destroying the lipid envelope of the virus.

In contrast to cationic, anionic, and ampholytic surface active agents, the nonionics contain no ionizable groups and have no surface charge. They depend upon their entire molecule for surface activity. Almost any hydrophobic compound which has in its structure a carboxy, hydroxy, amido or amino group with a free hydrogen attached to the nitrogen, can be reacted with ethylene oxide to form a nonionic surfactant. At least three groups of nonionic surfactants are recognized: a) those having an ether linkage between the hydrophilic and hydrophobic portions of the molecule, b) those having an ester or ether-ester linkage, and c) those having an amide linkage. Nonionic surfactants having at least one ether or amide linkage are preferred for purposes of the present invention. Examples of preferred nonionic surfactants include the following: nonyl-phenoxy-polyethoxy ethanol (Nonoxynol®-9), p-diisobutylphenoxy-polyethoxy ethanol(Triton® X-100), polyoxyethylene (10) oleyl ether (Brij®-97), and Onyx-ol® 345.

An effective dosage of an antiviral nonionic surface active agent for purposes of the present inventions comprises 0.1% to 20% by weight of the pharmaceutical composition. The preferred range is 1% to 5% by weight.

The balance of the pharmaceutical compositions comprises an inert, physiologically acceptable carrier. The carrier should not react with the active ingredients and not reduce their effectiveness. Suitable physiologically acceptable carriers include water, ethanol, polyethylene glycol, mineral oil, petrolatum and propylene glycol.

The pharmaceutical compositions are preferably administered in lotion, cream, oil, or emulsion formulations.

The following are examples of suitable formulations containing a nonionic surfactant and a human interferon:

| Pharmaceutical lotion | |
|---|---|
| propylene glycol | 24.75 ml. |
| triethanolamine | 1.00 ml. |
| water | 7.00 ml. |
| oleic acid | 1.50 gm. |
| polyethylene glycol monostearate | 10.50 gm. |
| silicon fluids | 10.00 ml. |
| carbopol-934 (2% mucilage) | 50.00 ml. |
| human leukocyte interferon | $10^6$—$10^8$ I.U. |

4

# 0 077 063

Pharmaceutical cream A

| | |
|---|---|
| white petrolatum | 41.00 g. |
| microcrystalline wax | 3.00 g. |
| fluid lanolin | 10.00 g. |
| sorbitan monooleate | 4.75 g. |
| polysorbate-80 | 0.25 g. |
| purified water | 41.00 g. |
| human leukocyte interferon | $10^6$—$10^8$ I.U. |

Pharmaceutical cream B

| | |
|---|---|
| spermaceti | 7.5% |
| white wax | 12.0% |
| mineral oil | 56.0% |
| sodium borate | 0.5% |
| sorbitan monooleate | 5.0% |
| water | 19.0% |
| human leukocyte interferon | $10^5$—$10^7$ I.U. |

Topical administration of pharmaceutical compositions of the present invention may be effected by applying a small amount (e.g., about 1 ml) of the compositions directly to and in areas adjacent to the site of the lesions with a cotton swab, soft brush, sponge or the like. Any quantity sufficient to cover the area of the lesions is effective. Treatment with the pharmaceutical compositions herein disclosed should be frequent, for example, every 2—4 hours, for about 3—4 days. Preferably, the pharmaceutical compositions are applied during an early stage of viral multiplication. For example, the compositions may be applied during the prodromal stage in the areas where a tingling sensation is felt. When the compositions are applied to lesions, the pain is substantially reduced within 1 hour in almost all cases. The lesions are noticeably improved within 4 to 12 hours, and are completely healed within 4 to 5 days with little or no scarring. When the compositions are applied during the prodromal stage, the tingling sensation usually disappears within the hour and the compositions provide a prophylaxis against emergence of the lesions. In some cases, no lesion appears at all; in other cases a small blister appears which disappears within 2—3 days. Additionally, treatment of herpes simplex virus infections in accordance with the present invention appears to reduce the frequency of recurrent attacks and in some cases apparently eliminates the virus altogether. No contraindications have been found.

The pharmaceutical compositions of the present invention also display antimicrobial activity as well as antiviral activity. For example, they are effective in treating warts as well as bacterial infections. As used herein, the term antimicrobial activity refers to activity against microorganisms other than viruses such as bacteria and fungi.

In addition to direct application, the pharmaceutical compositions may be administered topically by various other methods. For example, the compositions may be delivered to the affected region in microencapsulated form. They may also be delivered in a foam, by spray, tampon, vaginal suppository.

Although it is not completely understood, it is believed that the pharmaceutical compositions herein disclosed operate in the following manner. The antiviral nonionic surface active agent dissolves the lipid-containing envelopes of herpes simplex virus thereby destroying its infectivity. The interferon aids the cells in preventing viral replication. In addition, because very few interferon molecules are needed to protect a cell, the interferon prevents the spread of the virus to cells below the uppermost layer. Thus, the combination of a nonionic surface active agent, and human interferon is more effective against herpes simplex virus than would be expected by simply adding the effectiveness of the two active ingredients. It is believed that this synergistic effect retards recurrent attacks of the virus.

In another embodiment, this invention relates to a cosmetic composition containing $10^2$ to $10^8$ I.U. per dosage unit of a human interferon, an antiviral nonionic surface active agent, and a physiologically acceptable cosmetic carrier. Additional components, for example, skin softeners, may be included in the cosmetic formulations.

Cosmetic formulations are known in the art and are usually hypoallergenic and pH controlled. The cosmetic formulations of this invention are used as a prophylactic to prevent outbreak of herpetic lesions. They may be applied nightly. Cosmetic formulations according to the present invention generally contain less human interferon and antiviral nonionic surface active agent than pharmaceutical preparations. The preferred range of human interferon is $10^3$—$10^5$ I.U. and the preferred range of the antiviral nonionic surface active agent is 0.1% to 1% by weight in the composition. A typical cosmetic formulation according to the present invention is the following:

Cosmetic cream

| | |
|---|---|
| beeswax | 12.1% |
| spermaceti | 12.6% |
| sweet almond oil | 54.4% |
| borax | 0.5% |
| rose water | 19.4% |
| Onyx-ol® 345 | 1.0% |
| human leukocyte interferon | $10^3$—$10^5$ I.U. |

The following examples are presented in further illustration of the present invention:

Example 1

To determine whether human interferon is compatible with antiviral nonionic surface active agents, monolayers of human embryo cells were grown. Human leukocyte interferon ($10^5$ I.U.) was mixed with a 1% solution of nonylphenoxypolyethoxy ethanol (without a carrier) and with a proprietary cream formulation containing 1% nonylphenoxypolyethoxy ethanol (Stearox®). The interferon was kept in contact with the nonionic antiviral surfactant overnight and the mixture was then diluted 1:$10^4$ with a standard medium saline solution. The mixture was then applied to the monolayers of human embryo cells overnight at 37°C. The next day the human embryo cells were exposed to vesicular stomatitis virus and after a suitable period of time the cultures were assayed for virus plaques. The assays were compared to assays taken when the cells were exposed to vesicular stomatitis virus without the benefit of any antiviral agent and with the benefit of human leukocyte interferon alone (diluted as above). The results demonstrate that there is no reduction in activity of human interferon when combined with the nonionic surface active agent.

Example 2

The procedure of Example 1 was followed except that the human embryo cells were exposed to herpes simplex virus types 1 and 2 instead of vesicular atomatitis virus. The results demonstrate that human inteferon remains active against herpes simplex vurus in the presence of an antiviral nonionic surface active agent. The tests show that human interferon is compatible with an antiviral nonionic surface active agent.

Example 3

Patient A, a female, suffers recurrent herpes simplex virus infections of the face. Lesions erupt approximately every 3 to 4 months and cover most of her face. The next time she feels a tingling sensation indicating the prodromal stage of viral multiplication, she applies the pharmaceutical lotion described above directly to her entire face. The lotion is applied very 2 hours while she is awake for 3 days. The tingling sensation disappears within an hour after the first application. Two days after the first application, a small blister appears on her lip. The blister disappears a day later leaving no scar. Patent does not report any subsequent outbreaks.

Example 4

Patient B, a female suffers from recurrent attacks of herpes genitalis. On the average, outbreaks occur every 8 weeks and symtpoms last for about 2 weeks. She is treated with the pharmaceutical cream A described above during the prodromal stage of the next outbreak. The cream is applied very 4 hours for 3 days. The tingling sensation disappears almost immediately and no eruptions are subsequently observed. Recurrent outbreaks are also not reported.

Example 5

Patient C, a male, suffers from recurrent attacks of herpes genitalis. Eruptions appear every 3—4 months on the average and last about 10—14 days. At the next outbreak, a pharmaceutical cream B described above is applied as soon as the eruptions begin to appear. The pharmaceutical cream is applied every 2 hours. The eruptions are much smaller and fewer in number than usually appear and last for only 2 days. Patient C thereafter applies the cosmetic cream described above once a day. No subsequent outbreaks are reported.

The treatment of skin malignancies such as squamous cell carcinoma and basal cell carcinoma and premalignant lesions such as actinic keratosis and leukoplakia involves topical application of a pharmaceutical composition. containing an effective amount of a human interferon and an antiviral nonionic surface active agent, as described above. The topical administration could be accomplished with or without un occlusive dressing, but preferably with an occlusive dressing. The lesions are preferably treated daily for a period of about ten (10) days by topical administration of a pharmaceutical composition containing interferon and an antiviral nonionic surfactant. After such time, the lesions would be evaluated and a determination made whether further or continued treatment should be undertaken. For example, observation should be made for improvement in lesions or for possible new lesions.

The treatment is also useful in cases where squamous or basal cell carcinomas have been surgically

# 0 077 063

removed, but the tumor margins of the specimen showed tumor cells. The treatment would be carried out as a form of prophylactic therapy, with monitoring for recurrences.

Although ten (10) day courses of daily application with an occlusive dressing are preferable in the treatment of malignant and pre-malignant skin lesions, modifications can be readily made as necessary in treatment of specific cases.

In treatment of herpes zoster

topical application, preferably with an occlusive dressing, would be made of a pharmaceutical composition containing interferon and an antiviral nonionic surfactant, as described above. Application would be made two to three times daily until there was evidence of healing. By using the pharmaceutical composition of the present invention, pain associated with herpes zoster is alleviated because of local healing. Additionally, use of the pharmaceutical composition of the present invention in treatment of herpes zoster has been found to prevent postherpetic neuralgia.

The lesions associated with herpes zoster have been determined to heal faster than if left untreated.

In the treatment of psoriasis

repeated courses of topical application of interferon and an antiviral nonionic surfactant in a physiologically acceptable carrier according to the present invention would be required. Psoriasis is a chronic, recurring condition, for which there is no known cure. Since large areas of the skin are often involved, a plastic occlusive dressing would preferably be used to insure absorption into the skin of the pharmaceutical composition containing human interferon and an antiviral nonionic surfactant. Additionally, since large areas may be involved and hence require treatment, a significant amount of the human interferon would be absorbed into the bloodstream. Such may have a favorable effect on the systemic aspects of psoriasis, i.e., psoriatic arthritis.

Topical administration

may be effected by applying a small amount (e.g., about 1 ml) of the compositions containing human interferon and an antiviral nonionic surfactant directly to and in areas adjacent to the site of lesions with a cotton swab, soft brush or sponge. The quantity applied would be dependent on the size of the lesions being treated. Any quantity sufficient to cover the area of the lesions is effective.

In addition to direct application, the pharmaceutical compositions containing human interferon and an antiviral nonionic surfactant may be administered topically by various other methods. For example, the compositions may be delivered to the affected skin region in microencapsulated form. The pharmaceutical compositions may also be delivered in a foam or by spray.

As indicated hereinabove, the pharmaceutical compositions of this invention are also useful as a prophylactic for and in the treatment of *in situ* cervical carcinoma. In this application the interferon-containing compositions would be applied directly to the cervical or genital area.

## Claims

1. A pharmaceutical composition for treating herpes simplex viral infections, microbial skin infections, malignant skin lesions, pre-malignant skin lesions and/or skin lesions associated with herpes zoster or psoriasis in humans, consisting of $10^2$ to $10^8$ I.U. per dosage unit of a human interferon, an effective amount of an antiviral nonionic surface active agent, and a physiologically acceptable carrier for topical application.

2. The composition of Claim 1 wherein the amount of antiviral surface active agent is 0.1 to 20%, preferably 1% to 5%.

3. The composition of Claim 1 wherein the nonionic surface active agent has at least one ether or amide linkage.

4. The composition of Claim 3 wherein the nonionic surface active agent is nonylphenoxy-polyethoxy ethanol, p-diisobutylphenoxy-polyethoxy ethanol, polyoxyethylene (10) oleyl ether, or Onyx-ol® 345.

5. The composition of Claim 4 wherein the human interferon is leukocyte interferon.

6. The composition of Claim 5 wherein the amount of human interferon is $10^4$ to $10^6$ I.U.

7. The composition of Claim 1 wherein said carrier is a pharmaceutically acceptable carrier and/or a cosmetically acceptable carrier.

8. The composition of Claim 1 wherein the carrier includes water, ethanol, polyethylene glycol, mineral oil, petrolatum, or propylene glycol.

## Patentansprüche

1. Arzneimittel zur topischen Behandlung von Herpes simplex Virusinfektionen, mikrobiellen Hautinfektionen, malignen Hautdefekten, präkanzerösen Hautdefekten, und/oder mit Herpes zoster oder Psoriasis einhergehenden Hautdefekten beim Menschen, das aus $10^2$ bis $10^8$ I.E. pro Dosiseinheit menschlichem Interferon, einer wirksamen Menge eines antiviralen, nicht-ionischen oberflächenaktiven Mittels und einem physiologisch verträglichen Trägerstoff besteht.

2. Mittel nach Anspruch 1, in dem die Menge des oberflächenaktiven antiviralen Mittels 0.1 bis 20%, vorzugsweise 1 bis 5% beträgt.

3. Mittel nach Anspruch 1, in dem das nicht-ionische oberflächenaktive Mittel mindestens eine Äther- oder Amidbindung aufweist.

4. Mittel nach Anspruch 3, in dem das nicht-ionische oberflächenaktive Mittel Nonylphenoxy-Polyäthoxyäthanol, p-Diisobutylphenoxy-polyäthoxyäthanol, Polyoxyäthylen(10)oleyl äther, oder Onyx-ol® 345 ist.

5. Mittel nach Anspruch 4, in dem das menschliche Interferon Leukozyteninterferon ist.

6. Mittel nach Anspruch 5, in dem die Menge an menschlichem Interferon $10^4$ bis $10^6$ I.E. beträgt.

7. Mittel nach Anspruch 1, in dem der Trägerstoff ein pharmakologisch verträglicher und/oder kosmetisch verträglicher Trägerstoff ist.

8. Mittel nach Anspruch 1, in dem der Trägerstoff Wasser, Äthanol, Polyäthylenglykol, Mineralöl, Petrolatum oder Propylenglykol enthält.

## Revendications

1. Composition pharmaceutique pour le traitement des infections virales provoquées par l'herpès simple, des infections cutanées microbiennes, des lésions cutanées malignes, des lésions cutanées prémalignes et/ou des lésions cutanées associées à l'herpès zoster ou au psoriasis chez l'être humain, comprenant $10^2$ à $10^8$ U.I. par unité de dosage d'un interféron humain, une quantité efficace d'un agent tensio-actif non ionique antiviral et un support physiologiquement acceptable pour l'application topique.

2. Composition suivant la revendication 1, caractérisée en ce que la quantité d'agent tensio-actif antiviral est de 0,1 à 20%, de préférence de 1% à 5%.

3. Composition suivant la revendication 1, caractérisée en ce que l'agent tensio-actif non ionique comporte au moins une liaison éther ou amide.

4. Composition suivant la revendication 3, caractérisée en ce que l'agent tension-actif non ionique est le nonylphénoxypolyéthoxy éthanol, le p-diisobutylphénoxy-polyéthoxy éthanol, le polyoxyéthylène (10) oléyl éther ou l'Onyx-ol (marque déposée) 345.

5. Composition suivant la revendication 4, caractérisée en ce que l'interféron humain est de l'interféron de leucocyte.

6. Composition suivant la revendication 5, caractérisée en ce que la quantité d'interféron humain est de $10^4$ à $10^6$ U.I.

7. Composition suivant la revendication 1, caractérisée en ce que le support est un support pharmaceutiquement acceptable et/ou un support cosmétiquement acceptable.

8. Composition suivant la revendication 1, caractérisée en ce que le support comprend de l'eau, de l'éthanol, du polyéthylène glycol, de l'huile minérale, du pétrolatum ou du propylène glycol.